(19) Europäisches Patentamt
European Patent Office
Office européen des brevets

(11) Publication number : **0 404 807 B1**

(12) # EUROPEAN PATENT SPECIFICATION

(45) Date of publication of patent specification : **30.06.93 Bulletin 93/26**

(51) Int. Cl.$^5$ : **A61K 31/135,** A61L 15/16

(21) Application number : **89903719.6**

(22) Date of filing : **18.03.89**

(86) International application number : **PCT/EP89/00291**

(87) International publication number : **WO 89/09051 05.10.89 Gazette 89/24**

(54) **DEPRENYL FOR SYSTEMIC TRANSDERMAL ADMINISTRATION.**

(30) Priority : **29.03.88 GB 8807504**

(43) Date of publication of application : **02.01.91 Bulletin 91/01**

(45) Publication of the grant of the patent : **30.06.93 Bulletin 93/26**

(84) Designated Contracting States : **AT BE CH DE FR GB IT LI LU NL SE**

(56) References cited :
**EP-A- 241 809**
**EP-A- 0 139 127**
**GB-A- 2 163 347**
**US-A- 4 568 343**
**Martindale, The Extra Pharmacopoeia, 28th edition, 1982, edited by James E.F. Reynolds et al., publ. by The Pharmaceutical Press, (London, GB), pages 1752-1753 see pages 1752-1753, "Selegiline hydrochloride"**

(56) References cited :
**Psychopharmacol. Bull., volume 21, no. 3, 1985, C.R. Gardner: "Targeting the central nervous system: new drug delivery technologies for psychotropic agents", pages 657-662 see the whole article**
**Movement Disorders, volume 2, no. 3, 1987, Movement Disorder Society, W. Koller et al.: "PHNO, a novel dopamine agonist, in animal models of Parkinsonism", pages 193-199 see page 196**

(73) Proprietor : **SOMERSET PHARMACEUTICALS, INC.**
**777 S. Harbour Island Boulevard, Suite 880**
**Tampa, Florida 33602 (US)**

(72) Inventor : **KAROBATH, Manfred**
**Rebgasse 30**
**CH-4102 Binningen (CH)**
Inventor : **REINHARDT, Jörg**
**Schauinslandstrasse 5**
**W-7801 Ehrenkirchen (DE)**

(74) Representative : **Lightfoot, Robert Oscar et al**
**RAWORTH, MOSS & COOK, Raworth House,**
**36 Sydenham Road**
**Croydon, Surrey CR0 2EF (GB)**

EP 0 404 807 B1

Note : Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Beschreibung

The present invention provides the systemic transdermal application of deprenyl.

Deprenyl[N-methyl-N-(1-phenyl-2-propyl)-2-propinylamine] of formula I

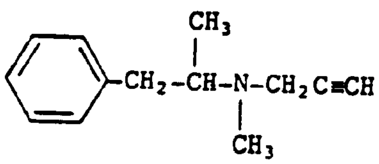

I

has been disclosed in the literature as a monoamine oxidase inhibitor. The preparation of the racemic mixture is described e.g. in fr. pat. M 2635, whereas Dutch Patent Application 6,605,956, for instance, discloses the (-)-form of the compound (also known as L-Deprenil, L-Deprenaline or Selegiline). The antidepressive and antiparkinson activity of the racemate and the (-)-form have been reported in various publications.

European Patent Application Serial No. EP 0241809 A discloses the use of a combination of deprenyl and amantadine as a synergistic combination for the treatment of Parkinson's disease. However this disclosure states that deprenyl alone exhibits almost no anti-Parkinsonism action; furthermore, given that the skin functions as a protective cover over the body, it cannot be assumed ab initio that deprenyl is suitable for transdermal administration.

It has now surprisingly been found that the compound of formula I in racemic or optically active form as well as the pharmaceutically acceptable acid addition salts thereof, hereinafter referred to as compounds for administration according to the invention, exhibit unexpectedly good skin penetration when administered percutaneously.

The penetration through the skin of the compounds for administration according to the invention may be observed in standard in vitro or in vivo tests.

One in vitro test is the well known diffusion test which may be effected according to the principles set out in GB 2098865 A and by T J Franz in J. Invest. Dermatol. (1975) 64, 194-195. The composition containing the active agent in unlabelled or radioactively labelled form is applied to one side of isolated pieces of intact human skin or hairless rat about 2 $cm^2$ in area. The other side of the skin is in contact with physiological saline. The amount of active agent in the saline is measured in conventional manner, e.g. by HPLC or spectrophotometric techniques, or by determining radioactivity.

In this test using rat skin the following penetration rates, for example, have been found:

| | | |
|---|---|---|
| Composition 1: | Compound of formula I in | |
| (solution) | (-)-form as hydrochloride | 35 mg |
| | Ethanol | 970 mg |
| | Polyol-polyether-fatty | |
| | acid ester, e.g. Cetiol HE* | 30 mg |

| | | | |
|---|---|---|---|
| Composition 2: | Compound of formula I | | |
| (polymer film) | in (-)-form as hydrochloride | 10 | % |
| | Hydroxypropylcellulose, | | |
| | e.g. Klucel LF* | 90 | % |

| | | | | |
|---|---|---|---|---|
| Composition 3: | Compound of formula I | | | |
| (solution) | in (-)-form as hydrochloride | | 7.3 | mg |
| | Ethanol | | 0.2 | ml |
| | Isopropylmyristate | ad | 1.0 | ml |

* : Registered Trade Mark

| | Penetration rates (24 h) | | | | | |
|---|---|---|---|---|---|---|
| | Receptor medium | | Skin | | Total | |
| | % | $mg/cm^2$ | % | $mg/cm^2$ | % | $mg/cm^2$ |
| Composition 1 | 5.2 | 0.267** | 1.3 | 0.067 | 6.5 | 0.334 |
| Composition 2 | 4.3 | 0.022 | 0 | 0 | 4.3 | 0.022 |
| Composition 3 | 36.1 | 0.434 | 8.2 | 0.098 | 44.3 | 0.532 |

**: Corresponds to $6.0 \times 10^{-8}$ $mole/cm^2/hour$

Moreover it has been found that transdermal administration of the compounds for administration according to the invention induces a long-lasting and constant inhibition of monoamine oxidase activity as indicated in standard tests, with a slow onset of action, which is particularly advantageous with respect to the tolerability of these compounds.

Thus the present invention provides a pharmaceutical composition for systemic transdermal administration, incorporating as sole active agent the compound of formula I in racemic or optically active form or a pharmaceutically acceptable acid addition salt thereof.

Preferably such a pharmaceutical composition has a penetration rate of at least $10^{-9}$ $mole/cm^2/h$, more preferably of at least $10^{-8}$ $mole/cm^2/h$. Suitably the penetration rate after 24 hours is at least 3%, preferably at least 10%.

The compound of formula I is preferably present in (-)-form and as hydrochloride.

In a further aspect the present invention provides the use of the compound of formula I in racemic or optically active form or a pharmaceutically acceptable acid addition salt thereof, as sole active agent in the manufacture of a pharmaceutical composition suitable for systemic transdermal administration.

The active agent may be administered in any conventional liquid or solid transdermal pharmaceutical composition, e. g. as described in Remington's Pharmaceutical Sciences 16th Edition Mack; Sucker, Fuchs and Spieser, Pharmazeutische Technologie 1st Edition, Springer and in GB 2098865 A or DOS 3212053.

Conveniently the composition is in the form of a viscous liquid, ointment or solid reservoir or matrix. For example the active agent is dispersed throughout a solid reservoir or matrix made of a gel or a solid polymer, e.g. a hydrophilic polymer as described in European Patent Application No. 155,229.

The active agent may be incorporated in a plaster.

The compositions for transdermal administration may contain from 1 to 50 % by weight of active agent.

The pharmaceutical compositions for transdermal administration may be used for the same indications as for oral or intravenous administration. The amount of pharmaceutically active agent to be administered will individually depend on the drug release characteristics of the pharmaceutical compositions, the drug penetration rate observed in in vitro and in vivo tests, the potency of active agent, the size of the skin contact area, the part of the body to which the unit is stuck, and the duration of action required. The amount of active agent and area of the pharmaceutical composition etc. may be determined by routine bioavailability tests comparing the blood levels of active agents after administration of the active agent in a pharmaceutical composition according to the invention to intact skin and blood levels of active agent observed after oral or intravenous administration of a therapeutically effective dose of the pharmacologically active agent.

Given the daily dose of a drug for oral administration, the choice of a suitable quantity of drug to be incorporated in a transdermal composition according to the invention will depend upon the pharmacokinetic properties of the active agent, taking into account that there is no first pass effect; the amount of drug which can be absorbed through the skin from the matrix in question for a given area of application and in a given time; and the time for which the composition is to be applied. Thus, a drug with a high first pass effect may require a relatively low quantity in the transdermal composition when compared with the oral daily dose, since the first pass effect will be avoided. On the other hand, generally a maximum of only approximately 50 % of the drug in the matrix is released through the skin in a 3 day period.

The pharmaceutical compositions of the invention in general have for example an effective contact area of drug reservoir on the skin of from 1 to 50 square centimetres, preferably about 2 to 20 square centimetres, and are intended to be applied for from 1 - 7 days, preferably 1 - 3 days.

Unit dosage forms preferably contain from 1 mg to 50 mg of the compound for administration according to the invention.

The compounds for administration according to the invention may for example be administered at a dose of 10 mg in a patch of ca. 10 $cm^2$, once every three days.

The compositions according to the invention may contain further active substances, e.go further agents which exhibit activity in the treatment of Parkinson's disease, such as levodopa or bromocriptine, or agents with antidepressive activity such as 1-phenylalanine. They may thus be used for the treatment of various conditions including Parkinson's disease and depression.

The compositions according to the invention are preferably used for the treatment of Parkinson's disease. The present invention thus more particularly provides a method of treating a subject suffering from Parkinson's disease, wherein a pharmaceutical composition of the invention is applied onto the skin.

The following example illustrates the invention.

EXAMPLE : Preparation of a transdermal composition containing a hydrophilic polymer

Composition

| | |
|---|---|
| Compound of formula I in (-)-form as hydrochloride | 20 % |
| Hydrophilic polymer, e.g. Eudragit E 100* | 30 % |
| Non swellable acrylate polymer, e.g. Durotack 280 - 2416** | 44 % |
| Plasticizer, e.g. Brij 97*** | 6 % |

* : Registered Trade Mark, available from Röhm, Darmstadt, W. Germany

** : Registered Trade Mark, available from Delft National Chemie Zutphen, Netherlands

***: Registered Trade Mark, available from Atlas Chemie, W. Germany

The components are added to acetone or ethanol or another appropriate volatile organic solvent and mixed to give a viscous mass. The mass is spread on top of an aluminised polyester foil (thickness 23 μm (microns)) using a conventional apparatus, to produce a film of thickness 0.2 mm when wet. The film is allowed to dry at

room temperature over 4 to 6 hours. The aluminium foil is then cut up into patches about 10 sq cm in area.

## Claims

1. A pharmaceutical composition for systemic transdermal administration, incorporating as sole active agent N-methyl-N-(1-phenyl-2-propyl)-2-propinylamine in racemic or optically active form or a pharmaceutically acceptable acid addition salt thereof.

2. A pharmaceutical composition according to claim 1, wherein the active agent is the hydrochloride of the (-)- form.

3. A pharmaceutical composition according to claim 1 comprising a transdermal patch structure.

4. The use of N-methyl-N-(1-phenyl-2-propyl)-2-propinylamine in racemic or optically active form or a pharmaceutically acceptable acid addition salt thereof, as sole active agent in the manufacture of a pharmaceutical composition suitable for systemic transdermal administration.

5. The use of the N-methyl-N-(1-phenyl-2-propyl)-2-propinylamine-hydrochloride, as sole active agent in the manufacture of a pharmaceutical composition according to claim 2.

6. The use of N-methyl-N-(1-phenyl-2-propyl)-2-propinylamine in racemic or optically active form or a pharmaceutically acceptable acid addition salt thereof, as sole active agent in the manufacture of a pharmaceutical composition suitable for systemic transdermal administration in the treatment of Parkinson's disease.

7. The use of the N-methyl-N-(1-phenyl-2-propyl)-2-propinylamine-hydrochloride, as sole active agent in the manufacture of a pharmaceutical composition according to claim 2, for the treatment of Parkinson's disease.

## Patentansprüche

1. Pharmazeutische Zusammensetzung zur systemischen transdermalen Verabreichung, enthaltend als einziges aktives Mittel N-Methyl-N-(1-phenyl-2-propyl)-2-propinylamin in racemischer oder optisch aktiver Form oder als pharmazeutisch verwendbares Säureadditionssalz davon.

2. Pharmazeutische Zusammensetzung nach Anspruch 1, worin das aktive Mittel das Hydrochlorid der (-)-Form ist.

3. Pharmazeutische Zusammensetzung nach Anspruch 1, enthaltend eine transdermale Pflasterstruktur.

4. Verwendung von N-Methyl-N-(1-phenyl-2-propyl)-2-propinylamin in racemischer oder optisch aktiver Form oder als ein pharmazeutisch verwendbares Säureadditionssalz davon als einziges Mittel zur Herstellung einer pharmazeutischen Zusammensetzung, die zur systemischen transdermalen Verabreichung geeignet ist.

5. Verwendung von N-Methyl-N-(1-phenyl-2-propyl)-2-propinylaminhydrochlorid als einziges aktives Mittel zur Herstellung einer pharmazeutischen Zusammensetzung nach Anspruch 2.

6. Verwendung von N-Methyl-N-(1-phenyl-2-propyl)-2-propinylamin in racemischer oder optisch aktiver Form oder als ein pharmazeutisch verwendbares Säureadditionssalz davon als einziges Mittel zur Herstellung einer pharmazeutischen Zusammensetzung, die geeignet ist zur systemischen transdermalen Verabreichung zur Behandlung der Parkinsonkrankheit.

7. Verwendung von N-Methyl-N-(1-phenyl-2-propyl)-2-propinylaminhydrochlorid als einziges aktives Mittel in der Herstellung einer pharmazeutischen Zusammensetzung gemäß Anspruch 2 zur Behandlung der Parkinsonkrankheit.

## Revendications

1. Composition pharmaceutique pour une administration transdermique systémique, comprenant, en tant qu'agent actif unique, de la N-méthyl-N-(phényl-1 propyl-2)-propinyl-2 amine, sous forme racémique ou optiquement active, ou un sel d'addition d'acide pharmaceutiquement acceptable de celle-ci.

2. Composition pharmaceutique selon la revendication 1, dans laquelle l'agent actif est le chlorhydrate de la forme (-).

3. Composition pharmaceutique selon la revendication 1, comprenant une structure d'emplâtre transdermique.

4. Utilisation de la N-méthyl-N-(phényl-1 propyl-2)-propinyl-2 amine sous forme racémique ou optiquement active, ou d'un sel d'addition d'acide pharmaceutiquement acceptable de celle-ci, en tant qu'agent actif unique dans la fabrication d'une composition pharmaceutique appropriée pour une administration transdermique systémique.

5. Utilisation du chlorhydrate de N-méthyl-N-(phényl-1 propyl-2)-propinyl-2 amine, en tant qu'agent actif unique dans la fabrication d'une composition pharmaceutique selon la revendication 2.

6. Utilisation de la N-méthyl-N-(phényl-1 propyl-2)-propinyl-2 amine sous forme racémique ou optiquement active, ou d'un sel d'addition d'acide pharmaceutiquement acceptable de celle-ci, en tant qu'agent actif unique dans la fabrication d'une composition pharmaceutique appropriée pour une administration transdermique systémique dans le traitement de la maladie de Parkinson.

7. Utilisation du chlorhydrate de N-méthyl-N-(phényl-1 propyl-2)-propinyl-2 amine, en tant qu'agent actif unique dans la fabrication d'une composition pharmaceutique selon la revendication 2, pour le traitement de la maladie de Parkinson.